Europäisches Patentamt

European Patent Office

Office européen des brevets

(11) Publication number: 0 324 969
A2

# EUROPEAN PATENT APPLICATION

(21) Application number: 88121529.7

(22) Date of filing: 22.12.88

(51) Int. Cl.⁴: G01N 33/68 , G01N 33/577 , C12P 21/00 , C12N 15/00

The title of the invention has been amended (Guidelines for Examination in the EPO, A-III, 7.3).

(30) Priority: 23.12.87 JP 323769/87

(43) Date of publication of application:
26.07.89 Bulletin 89/30

(84) Designated Contracting States:
BE DE FR GB IT NL

(71) Applicant: Tosoh Corporation
4560, Oaza Tonda
Shinnanyo-shi Yamaguchi-ken(JP)

(72) Inventor: Inouye, Kuniyo
37-17-402, Sagamiono 7-chome
Sagamihara-shi Kanagawa(JP)
Inventor: Murata, Shigeho
5-23-108, Soshigaya 2-chome Setagaya-ku
Tokyo(JP)
Inventor: Gohzu, Shunichi
35-21, Sakuradai Midori-ku
Yokohama-shi Kanagawa(JP)

(74) Representative: Vossius & Partner
Siebertstrasse 4 P.O. Box 86 07 67
D-8000 München 86(DE)

(54) Method of measuring transferrin.

(57) Transferrin in a sample is measured by sandwich assay or a competitive binding assay wherein a monoclonal antibody specifically recognizing transferrin is used.

EP 0 324 969 A2

# MEASUREMENT OF TRANSFERRIN

The present invention relates to a monoclonal antibody specifically recognizing transferrin and a method of measuring transferrin in a sample using the monoclonal antibody.

Transferrin is a protein having a molecular weight of 75,000 and having a capacity of binding with iron transferred in blood, which is called "siderophilin" or "iron-binding globulin". Transferrin is present in the serum of a normal adult in an amount capable of binding with 2 to 3 $\mu$g/ml of iron, and this amount is called "total iron binding capacity". Transferrin binds with iron in an amount corresponding to about 1·3 of this amount. That is, transferrin binds with 0.8 to 1.5 $\mu$g/ml of iron. The amount of remaining transferrin not binding with iron is called "unsaturated iron binding capacity" (hereinafter referring to as "UIBC").

Juvenile erythrocytes have an attraction to iron bound to transferrin, and in order for serum iron to be utilized for the synthesis of hemoglobin, serum iron must be bound to transferrin.

Transferrin is sensitive to a stored iron level. Therefore, transferrin is an important parameter for iron metabolism and hemogenesis. Furthermore, transferrin is a substance important for the propagation of cells and for biological defence, and reflects various disease states. Increase of transferrin in blood indicates a decrease of stored iron, which occurs with the progress of pregnancy. Transferrin also is increased in the case of iron-deficient anemia, acute hepatitis or acute liver trouble. Reduction of transferrin in blood indicates an increase of stored iron. As the disease in which the reduction of transferrin is observed very frequently, there can be mentioned various malignant tumors, inflammation, blood diseases, hepatocirrhosis, unbalanced nutrition, protein-transducing enteritis, kidney diseases and inherited atransferrinemia.

Various methods have heretofore been proposed for measuring transferrin in blood. For example, iron in an amount sufficient to cover UIBC is added to serum to saturate the transferrin in the serum with iron, and the iron ion left unbound to UIBC is removed by adsorption on an iron-adsorbing material. Then, the amount of iron is measured, as in the case of the measurement of serum iron according to the colorimetric method, the electrode method or the atomic-absorption spectroscopic method. A specific measurement apparatus is necessary for each of these methods.

The immunochemical measurement method utilizing an antibody to transferrin has been reported (Journal of Japanese Clinical Association, autumn special edition, No. 1, page 546, 1985). According to this proposal, transferrin is measured by immunodiffusion, radioimmunoassay or turbidimetry. These measurement methods have problems in that the measurement time is long and the operation is complicated.

It is an object of the present invention to solve the above-mentioned problems and to provide a method of measuring transferrin using a monoclonal antibody specific to transferrin, in which transferrin can be measured very simply and at a high sensitivity.

In one aspect of the present invention, there is provided a monoclonal antibody specifically recognizing transferrin.

In another aspect of the present invention, there is provided a method of measuring transferrin in a sample, in which a monoclonal antibody specifically recognizing transferrin is used.

The monoclonal antibody of the present invention is preferably obtained by collecting a monoclonal antibody recognizing transferring from a cultured product of hybridomas obtained by fusing an antibody-producing cell of an animal immunized with transferrin, to a myeloma cell to obtain a hybridoma producing a monoclonal antibody recognizing transferrin, and culturing the obtained hybridoma and/or a cell strain derived therefrom. The hybridoma producing the monoclonal antibody recognizing transferrin is prepared by a known cell-fusing method (G. Kohler and C. Milstein, Nature, 256, page 495, 1975). In the present invention, a plurality of monoclonal antibodies recognizing transferrin can be obtained according to the above-mentioned method. Part of the thus-obtained monoclonal antibodies have a binding constant larger than $10^8 M^{-1}$. By using the monoclonal antibodies having such a large binding constant, the measurement of transferrin can be effected at a higher sensitivity. It has been found that these monoclonal antibodies recognize different sites of transferrin.

The immunoassay of transferrin using such monoclonal antibodies capable of binding specifically with transferrin can be effected by the sandwich method or competitive method. For example, in the immunoassay according to the sandwich method, a known method can be adopted for immobilizing the antibody on a solid carrier. For example, as the solid carrier, there are preferably used microtiter plates or particles of glass, polystyrene, polyethylene, polyvinyl chloride, latex, agarose, celulose and methacrylate polymer. The labelling method for producing an antibody labelled with a marker or the method for detecting the thus labelled antibody is not particularly critical, and labelling and detection can be performed according to known methods. In the method using an enzyme as the marker (EIA), enzymes such as peroxidase, $\beta$-D-galactosidase and alkaline phosphatase can be used. In the method using a radioactive substance (RIA),

$^{125}I$ and $^3H$ can be used. In the method using a fluorescent substance (FIA), fluorescamine and fluorescein isothiocyanate can be used. Other markers can be used. Where the marker is an enzyme, a substrate is use for measuring the activity of the enzyme. For example, as the substrate for peroxidase, there can be mentioned 2.2'-adino-di-[3-ethylbenzthiazoline-6-sulfonic acid] diammonium salt (hereinafter referred to as "ABTS")-$H_2O_2$, 5-aminosalicylic acid-$H_2O_2$ and o-phenylenediamine-$H_2O_2$. As the substrate for $\beta$-D-galactosidase, there can be mentioned o-nitrophenyl-$\beta$-D-galactopyridinose, and as the substrate for alkaline phosphatase, there can be mentioned p-nitrophenyl phosphate. For the measurement, known reagents such as dissolving agents, washing agents and reaction stopper agents can be used in addition to the above-mentioned reagents.

In the method of the present invention using a monoclonal antibody specifically recognizing transferrin, transferrin can be measured at a concentration of 1 to 320 ng/ml in a sample, and only a very small amount of a sample is necessary. Moreover, as compared with the conventional method, the operation is very simple, and many samples can be tested in a short time according to this method.

The present invention will now be described in detail with reference to the following example that by no means limits the scope of the invention.

### Example 1

(1) A cell producing a monoclonal antibody recognizing transferrin was obtained by fusing an antibody-producing cell of a mouse immunized with transferrin to a myeloma cell, a cell strain derived from the thus-obtained hybridoma was cultured, and a monoclonal antibody recognizing transferrin was collected from the culture product.

(2) To each well of a 96-well microtiter plate (immunoplate supplied by NUNC) was added 250 $\mu$l of a solution of 1 $\mu$g/ml of a mouse monoclonal antibody to transferrin (hereinafter referred to as "antibody A") in 0.1M sodium carbonate buffer (pH = 9.6), and incubation was carried out at 4°C overnight. The solution was removed from each well, and each well was washed three times with phosphate-buffered physiological saline (0.01 M phosphoric acid buffer containing 0.85 % of NaCl, pH = 7.0; hereinafter referred to as "PBS") containing 0.04% of Tween 20 (hereinafter referred to as "PBS-T"). Then, 300 $\mu$l of a PBS-T solution containing 1% of BSA (bovine serum albumin) was added to each well, the blocking treatment was carried out at 4°C, and the microtiter plate was stored in this state at 4°C.

(3) Preparation of Antibody Labelled with Horseradish Peroxidase (hereinafter referred to as "HRP")

To 1 ml of a solution of 5 mg/ml of HRP in 0.3M sodium bicarbonate buffer (pH = 8.1) was added 0.1 ml of a 1 % solution of 1-fluoro-2,4-dinitrobenzene in ethanol, and reaction was carried out at room temperature for 1 hour. Then, 1.0 ml of 0.06M sodium periodate was added and the reaction was carried out for 30 minutes. Unreacted sodium periodate was removed by the addition of 1.0 ml of 0.16M ethylene glycol, and the reaction mixture was dialyzed against 0.01M sodium carbonate buffer (pH = 9.5).

Then, 5 mg of a mouse monoclonal antibody to transferrin (hereinafter referred to as "antibody B", recognizing a site different from the site recognized by the monoclonal antibody A) was added to the reaction mixture, and the reaction was carried out for 5 hours. Then, 5 mg of sodium borohydride was added to the reaction mixture, and the mixture was allowed to stand at 4°C overnight.

The thus-obtained reaction product was purified by the high-performance liquid chromatography using TSK Gel G-3000SW (trademark for the product supplied by Tosoh Corp.), whereby an HRP-labelled antibody was obtained.

(4) Measurement of Transferrin in Sample by Enzyme Immunoassay

The monoclonal antibody-immobilized microtiter plate prepared in (2) of the present example was returned to room temperature, and the microtiter plate was washed three times with PBS-T. Then, 20 $\mu$l of normal rabbit serum containing 1 to 320 ng/ml of refined transferrin was added to each well. Then, 4.2 mg/ml of the HRP-labelled antibody was diluted 1000 times with the PBS-T solution containing 1.5% of normal rabbit serum, 0.0075% of myoglobin, 0.0075% of refined white sugar and 0.06% of egg albumin (hereinafter referred to as "PBS-T solution for antigen-antibody reaction"), 300 $\mu$l of the dilution was added to each well, and incubation was carried out for 3 hours. Then, the solution was removed and each well was

3

washed three times with the PBS-T solution. Then, a substrate solution comprising 0.1M citrate buffer (pH = 4.1) containing 0.03% of ABTS and 0.01% of $H_2O_2$ was added to each well, and the reaction was carried out at room temperature for 30 minutes. The reaction was stopped by the addition of 100 μl of 1M oxalic acid solution. With respect to each well, the absorption intensity at 415 nm and the absorption intensity at a reference wavelength of 492 nm were measured by an automatic microtiter plate reader (MPR-A4 supplied by Tosoh Corp.). The concentration of the reference substance and the absorption intensity were plotted to obtain the results shown in Table 1 and a calibration curve was prepared.

Table 1

| Transferrin Concentration (ng/ml) | Absorbance (Abs) (measurement wavelength = 415 nm, reference wavelength = 492 nm) |
|---|---|
| 1.00 | 0.01 |
| 2.50 | 0.02 |
| 5.00 | 0.07 |
| 10.0 | 0.25 |
| 20.0 | 0.44 |
| 40.0 | 0.85 |
| 80.0 | 1.45 |
| 160 | 2.11 |
| 320 | 2.55 |

## Claims

1. A method of measuring transferrin in a sample, comprising using a monoclonal antibody specifically recognizing transferrin.

2. The method according to claim 1, wherein the monoclonal antibody specifically recognizing transferrin is prepared by fusing an antibody-producing cell of an animal immunized with transferrin to a myeloma cell to obtain hybridoma producing a monoclonal antibody recognizing transferrin, culturing the hybridoma or a cell strain derived therefrom, and collecting the thus-produced monoclonal antibody from the cultured product.

3. A monoclonal antibody specifically recognizing transferrin.